(19) Europäisches Patentamt
European Patent Office
Office européen des brevets

(11) **EP 3 002 696 B1**

(12) **EUROPEAN PATENT SPECIFICATION**

(45) Date of publication and mention
of the grant of the patent:
**06.03.2019  Bulletin 2019/10**

(51) Int Cl.:
**G06F 19/18** (2011.01)    **G06F 19/00** (2018.01)

(21) Application number: **15188001.0**

(22) Date of filing: **01.10.2015**

(54) **METHODS FOR GENERATING, SEARCHING AND STATISTICALLY VALIDATING A PEPTIDE FRAGMENT ION LIBRARY**

VERFAHREN ZUM ERZEUGEN, DURCHSUCHEN UND STATISTISCHEN VALIDIEREN EINER PEPTIDFRAGMENT-IONEN-BIBLIOTHEK

PROCÉDÉS POUR GÉNÉRER, RECHERCHER ET VALIDER STATISTIQUEMENT UNE BIBLIOTHÈQUE D'IONS DE FRAGMENT PEPTIDIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.10.2014  US 201462059621 P**

(43) Date of publication of application:
**06.04.2016  Bulletin 2016/14**

(73) Proprietor: **Thermo Finnigan LLC**
**San Jose, CA 95134 (US)**

(72) Inventor: **MOHTASHEMI, Iman**
**Mountain House, CA 95391 (US)**

(74) Representative: **Boult Wade Tennant LLP**
**Verulam Gardens**
**70 Gray's Inn Road**
**London WC1X 8BT (GB)**

(56) References cited:
**US-A1- 2014 138 535    US-A1- 2014 138 537**

**Description**

FIELD OF THE INVENTION

The present invention relates to methods of and systems for obtaining and analyzing spectra of ion products generated from one or more precursor ions. More particularly, the present invention relates to generation, searching and statistical validation of tandem mass spectra, subsequent to data acquisition, using local mass spectral libraries.

BACKGROUND OF THE INVENTION

Structural elucidation of ionized molecules of complex structure, such as proteins, is often carried out using a tandem mass spectrometer, where particular precursor ions are selected at the first stage of analysis or in a first mass analyzer, the precursor ions are subjected to fragmentation (e.g., in a collision cell), and the resulting fragment (product) ions are transported for analysis in the second stage or second mass analyzer. The method can be extended to provide fragmentation of a selected fragment, and so on, with analysis of the resulting fragments for each generation. This is typically referred to an $MS^n$ spectrometry, with n indicating the number of steps of mass analysis and the number of generations of ions. Accordingly, $MS^2$ mass analysis (also known as an MS/MS mass analysis) corresponds to two stages of mass analysis with two generations of ions analyzed (precursor and products). A resulting product spectrum exhibits a set of fragmentation peaks (a fragment set) which, in many instances, may be used as a fingerprint to derive structural information relating to the parent peptide or protein.

Some mass spectrometer systems will generally generate $MS^2$ spectral data in which precursor ions are not isolated prior to fragmentation and thus precursor ions of comprising a plurality of mass-to-charge ratios are simultaneously fragmented. Such systems generate data that may be described as "intentionally multiplexed" because the experimental results comprise, by design, multiple overlapping sets of fragment-ion data relating to multiple precursor ion types. Also, many conventional triple-quadrupole and other types of mass spectrometer systems may be operated such that they generate multiplexed $MS^2$ spectral data in an unintentional sense, as a result of imperfect isolation of certain precursor ions of a single *m/z* ratio or ratio range prior to fragmentation. Multiplexing may thus be said to be unintentional when a single isolation window (e.g. 1 Da) happens to contain multiple precursor ions in addition to the one being targeted for selection and fragmentation. The presence of multiplexing could be known or unknown. Regardless of whether multiplexing is intentional or unintentional, or known or unknown, there is a requirement for methods to that can resolve the various multiplexed components.

An $MS^2$ spectrum (a spectrum of fragment or product ions) can provide rich information about the covalent structure of an isolated precursor molecule. Unfortunately, despite the apparently very high information content of $MS^2$ spectra, the success rate for identifying molecules is very low. A number of factors may explain the low success rate, including incomplete or poorly curated databases and inadequate software. In proteomics, traditional database searching techniques rely on comparing experimental spectra of proteolytic-enzyme-digested proteins against computer-generated theoretical spectra comprising calculated peptide mass or fragment ion mass values obtained by applying cleavage rules to a comprehensive protein database. However, since not all databases are complete, experimental spectra of mutant or novel sequences not in a database, are missed in the identification schemes. This is, in part, responsible to the low identification (at best 50%) of $MS^2$ spectra are assigned a sequence. Further, conventional approaches to interpreting product ion spectra consider only the masses of the product ions, and not their relative intensities. Intensity information has been excluded from conventional analyses because it is difficult to predict relative product ion intensities *de novo,* especially since relative intensities of mass spectral linescan vary between instruments and according to experimental conditions. Still further, conventional database searching techniques, as described above, often do not detect the multiplexed spectra and only report the highest scoring match.

Spectral library searching may be considered as an alternative or as a complement to database searching, as described above. In this second approach, no theoretical MS/MS spectral generation and searching is necessary, the library comprises actual experimentally determined mass spectra. In this approach, where the product ion spectra of all potential precursors are assumed to be stored in a library, it is not necessary to predict intensities. United States Pre-Grant publications 2014/0138535 A1 and 2014/0138535 A1, both assigned to the assignee of this application and incorporated by reference herein in their entireties, describe generation and use of local mass spectral libraries, in which each such local library comprises spectral information obtained from a single mass spectral instrument and is used to identify spectra subsequently obtained on the same instrument. The local mass spectral libraries so described comprise information relating to both the mass-to-charge ratio and intensity of each observed mass spectral line. A requirement for employing such local mass spectral libraries is that product ion intensities be reproducible. On modern instruments, where acquisition parameters such as collision gas pressure and collision energy are standardized, product ion intensities are highly reproducible. High reproducibility allows quantitatively accurate interpretation of mixed product ion spectra. The inventors of U.S. Pre-Grant Publications 2014/0138535 A1 and 2014/0138535 A1 have discovered that the product- or fragment-ion intensities provide a very significant amount of information and are quite reproducible on a given instrument using invariant (i.e. standardized) acquisition parameters. Unfortunately, the use of local mass spectral libraries lacks the automatic species assignment information that may be provided by the use of conventional searching techniques.

One open question regarding the use of local mass spectral libraries pertains to the question of initially populating the library, for instance, at the time that a new mass spectral instrument is first installed and used. One obvious way of performing the initial population of library entries would be to experimentally determine the $MS^2$ spectra of a plurality of purified or chromatographically separated analyte compounds, one at a time, on the single mass spectral instrument, thereby deriving a series of standard spectra. Unfortunately, at least for the field of proteomics, the large number of possible peptide or polypeptide compounds that would need to be investigated in this fashion renders such an inefficient approach impractical. Another question regarding the use of local mass spectral libraries relates to the fact that rigorous statistical tests have not been described for the purposes of testing the statistical validity of an individual spectral decomposition, the statistical validity of various abundance coefficients arising from the decomposition, or the statistical validity of recognizing a previously unobserved component. The present disclosure addresses both of the above questions and also provides the capability of automatically including species assignments in the library at the time of its generation or modification.

SUMMARY OF THE INVENTION

Various aspects and embodiments of the invention are set out in the appended claim set.

In light of the above discussion, there is hereafter described a workflow and the corresponding mathematical principles for generating, searching and statistical validation of fragment ion spectra subsequent to data acquisition. The method applies quality control measures to generate a high quality peptide spectral library and applies a multiple regression model to identify and score new experimental data against the generated library. With regard to local mass spectral library generation, the mass spectrometer is operated in conventional fashion, so as to detect and record the mass spectra of peptide

and polypeptide compounds (possibly multiplexed) in mixtures of possibly unknown analytes - instead of in purified form. Once a number of such spectra have been obtained, conventional database searches, using commercially available software such as SEQUEST™ or MASCOT™ are conducted. The top scoring matches resulting from these searches are binned and zero-filled and then entered into the local mass spectral library.

Without regard to identification, a general library searching is proposed using a multiple regression technique. This problem is solved using the matrix inversion technique since all spectra can be expressed as a linear combination. The regression model is generated where each coefficient in the model represents a spectrum in the database that is constrained by precursor mass of arbitrary tolerance. Although matrix inversion can be used for computing the estimators, because it is a multiple regression model, a statistical f-test can be computed to estimate the model's validity. If the f-test passes, it indicates that at least one or more of the coefficients are statistically significant. Each coefficient can also be tested in terms of its statistical contribution to the model's validity. Since the spectrum is represented as a linear combination, if there are multiple coefficients that are statistically significant, then such a result is indicative of multiplexed spectra. Therefore, the method has the capability to identify multiplexed spectra from co-isolated precursors independent of precursor and product ion intensity covariance.

Accordingly, a linear-algebraic approach to analyzing and interpreting multiplex $MS^2$ spectra (fragment- or product-ion spectra) using a local mass spectral library is herein disclosed. Also disclosed is a method for automatically constructing a local spectral library.

As the term is used herein, a "multiplex" $MS^2$ spectrum contains fragments that arise from a mixture of precursors, in contrast to a "pure" $MS^2$ spectrum in which the fragments come from a single isolated precursor. Further, in the context of this disclosure, "spectral interpretation" means estimating the relative abundance of each precursor represented in the multiplex spectrum. The precursors are assumed to be represented within entries in a database of observed $MS^2$ spectra. Most product ion spectra are interpreted as linear superpositions of library spectra; however, some product ion spectra contain contributions from previously unrecorded precursors that can also be discovered during analysis.

Acquisition of multiplexed product ion spectra may be intentional or unintentional. In some cases, multiple precursors may isolated and combined prior to fragmentation so as to exploit the channel bandwidth of a high-resolution mass-analyzer so as to increase analytical throughput. Multiplexing may be said to be unintentional when a single isolation window (e.g. 1 Da) happens to contains multiple precursor ions in addition to the one being targeted for selection and fragmentation. These additional precursors may be below the limit of detection in an $MS^1$ (precursor ion) spectrum and yet be detectable in $MS^2$ spectra as a consequence of isolation because isolating narrow mass ranges typically involves significantly longer ion accumulation. Alternatively, a single detected peak in an $MS^1$ spectrum may be hiding multiple precursors with mass differences too small to be resolved, or in fact, structural isomers of identical mass. Therefore, the methods described herein make no assumption about the number of precursors. Instead, all candidate precursors within a given mass range are assumed to be present and their intensities (most often zero) are estimated. If, in fact, only one precursor is represented in the MS$^2$ spectrum, the algorithm will work as expected: identifying that precursor by assigning (essentially) zero abundance to all other candidates.

Optimal estimates of precursor abundances are determined from an observed MS$^2$ spectrum by solving a linear matrix equation. Typically, only a few precursor ion candidates are present.

BRIEF DESCRIPTION OF THE DRAWINGS

The above noted and various other aspects of the present invention will become apparent from the following description which is given by way of example only and with reference to the accompanying drawings, not drawn to scale, in which:

FIG. 1 is a schematic illustration of the occurrence of a mixture of precursor ions in an MS$^1$ spectrum, where the presence of multiple precursors may be undetected, giving rise to a multiplexed product ion spectrum;

FIG. 2 is a flow diagram of a method for populating a local mass spectral library in accordance with the present teachings; and

FIG. 3 is a flow diagram of an example method for recognizing or identifying measured mass spectra.

DETAILED DESCRIPTION

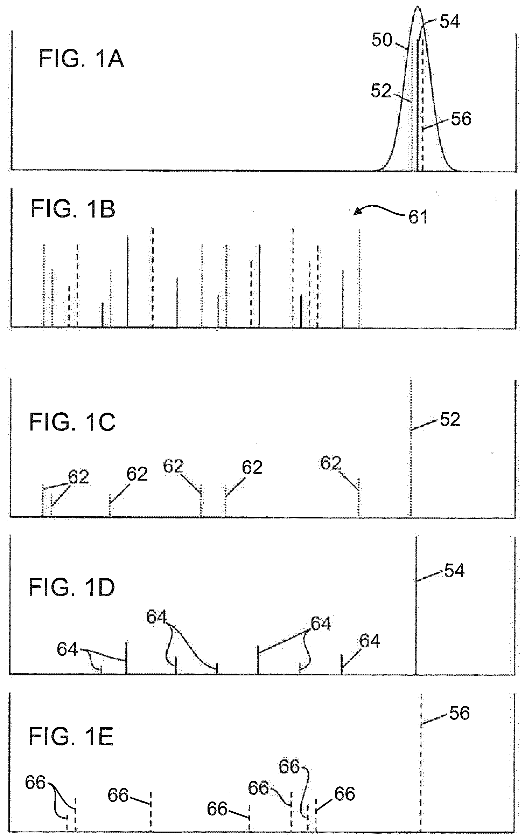

The following description is presented to enable any person skilled in the art to make and use the invention, and is provided in the context of a particular application and its requirements. Various modifications to the described embodiments will be readily apparent to those skilled in the art and the generic principles herein may be applied to other embodiments. Thus, the present invention is not intended to be limited to the embodiments and examples shown but is to be accorded the widest possible scope in accordance with the features and principles shown and described. To fully appreciate the features of the present invention in greater detail, please refer to FIGS. 1A-1E, 2 and 3 in conjunction with the following discussion.

*General Formulation*

This invention solves the problem of generating high quality experimental peptide fragment ion spectra while proposing searching and scoring technique of that library against newly acquired experimental spectra. Since library generation relies on statistical processing of database searching methods, this method further prunes all misidentified sequences generating a high quality library. It further does not need a reverse database since the multiple regression technique allows one to test the significance of all coefficients individually based on the variance. Since the library can be expressed as a linear combination this method can also detect multiplexed fragment ion spectrum independent of elution precursor and product ion intensity covariance. All precursors that coisolated can be assigned a fragment ion spectrum.

FIGS. 1A-1E schematically illustrate a typical problem that may occur during acquisition of a multiplex MS$^2$ spectrum. FIG. 1A illustrates a precursor ion spectrum (MS$^1$ spectrum) that may be obtained within a mass-to-charge envelope or window **50.** The envelope or window may comprise an isolation window or transmission window that is utilized so as to filter an initial plurality of ions such that only ions having mass-to-charge (*m/z*) values encompassed by the window are transmitted to a mass analyzer. Because the envelope or window **50** necessarily comprises a non-zero width, ions comprising more than one *m/z* ratio may be included in the precursor ion spectrum. The inclusion of more than one precursor *m/z* ratio may be either intentional or unintentional as described above.

In the hypothetical example illustrated in FIG. 1A, three different precursor ion types having different *m/z* ratios, represented by the lines **52, 54** and **56,** are included in the population of ions represented by the MS$^1$ spectrum. Simultaneous fragmentation of the precursor ion types produces fragment ions of different respective *m/z* ratios, as are represented by the lines **61** shown in the hypothetical MS$^2$ spectrum of FIG. 1B. Because three different precursor ion types are fragmented, the spectrum illustrated in FIG. 1B is a multiplex MS$^2$ spectrum that comprises first, second and third sub-populations of fragment ions whose various *m/z* ratios are represented, respectively, by dotted lines **62,** solid lines **64** and dashed lines **66.** The problem that is addressed by the present teachings is the recognition of the three different sub-populations of fragment ions and the association of each of the fragment ion sub-populations with a respective precursor ion type. For example, in FIGS. 1C, 1D and 1E, the fragment lines **62** are recognized as a first sub-population and associated with the precursor line **52,** the fragment lines **64** are recognized as a second sub-population and associated with the precursor line **54,** and the fragment lines **66** are recognized as a third sub-population and associated with the precursor line **56,** respectively.

In the novel algorithms described herein, a mathematical model for multiplex MS$^2$ spectra is presented and practical implementation of the model is described. The model for a product ion spectrum resulting from a mixture of precursors is a corresponding mixture of product ion spectra from the isolated precursors. This model has the following linearity property. Let M denote a mixture of precursors A, B, and C in the following proportions: $\beta_A$ parts of A, $\beta_B$ parts of B, and $\beta_C$ parts of C. The following notation is used to represent this mixture: $M = \beta_A A + \beta_B B + \beta_C C$. Let s(M) denote the "ideal" product ion spectrum obtained from X. In our model, $s(M) = \beta_A s(A) + \beta_B s(B) + \beta_C s(C)$, where s(A), s(B), and s(C) represent "ideal" product ion spectra that would be obtained from isolated (pure) precursors A, B and C. Each of the "ideal" product ion scans can be thought of as a vector, and so multiplication and addition have their usual intuitive meanings in the above equation.

Now suppose that a product ion spectrum s has been measured. According to the model analysis, the product ion spectrum is to be interpreted as a mixture of precursors A, B, and C. Thus, suppose that s = s(M), where $M = \beta_A A + \beta_B B + \beta_C C$, i.e. an arbitrary mixture of these precursors. In this case, ideal product ion spectra s(A), s(B), and s(C) are unavailable. Instead, it is here assumed that that *previously measured* product ion spectra of precursors A, B, C are available, which are denoted by d(A), d(B), d(C). The symbol d is here used to suggest that these product ion spectra reside in a local database. A novel aspect of the present teachings is that the entries of the local database are a filtered selection of spectra already obtained on the same mass spectrometer used to acquire the spectrum, s, wherein the filtering comprises entering, into the local database, observed sets of mass spectral lines that match entries in a conventional public database. The matches may be recognized using a conventional database search program, such as SEQUEST or MASCOT.

Given d(A), d(B), and d(C), one can mathematically generate arbitrary mixtures of product ion spectra: $s' = \beta'_A d(A) + \beta'_B d(B) + \beta'_C d(C)$, by choosing arbitrary values for coefficients $\beta'_A$, $\beta'_B$, and $\beta'_C$. Specifically, it is desired to find coefficients $\beta'_A$, $\beta'_B$, and $\beta'_C$ that minimize the difference between observed product ion spectrum s and the model mixture spectrum s'. Considering s and s' to be vectors (now denoted in vector notation as **s** and **s'**), the difference has the geometric interpretation as the length of the difference between two vectors. Geometrically, the set of all vectors **s'** that can be produced from d(A), d(B), and d(C) can be thought of as a hyperplane, where each set $(\beta'_A, \beta'_B, \beta'_C)$ identifies a point in this hyperplane. Let $(\hat{\beta}_A, \hat{\beta}_B, \hat{\beta}_C)$ denote the optimal values of $(\beta'_A, \beta'_B, \beta'_C)$, the coefficient values that minimize the difference between s and s'. According to the model, these optimal coefficients represent the estimates of the coefficients that characterize unknown mixture M that gave rise to product-ion spectrum **s** as a mixture of precursors A, B, and C. The value of $(\beta'_A, \beta'_B, \beta'_C)$ is simply the projection of **s** onto the hyperplane determined by vectors d(A), d(B), d(C) and can be calculated by solving a linear matrix equation as shown below.

In general, a mass spectrometer user is frequently presented with a product ion spectrum derived from a mixture of precursor ions. The mixture of precursor ions may be generated by applying a hardware filter, such as a quadrupole mass filter, that selects all ions in a given mass range. These ions can be indirectly visualized in a precursor spectrum (e.g., window **50** of FIG. 1A). The precursor spectrum typically covers the entire mass range and thus the ion capacity of the mass analyzer is allocated to the entire spectrum of ions. In the product ion scan, a fragmentation cell is filled only with ions in the selected mass range (isolation window) and these ions are subsequently injected into the mass analyzer. By devoting the entire capacity of the analyzer to this relatively narrow mass range, it is possible to detect ion species in a product ion spectrum that were not detected in the MS$^1$ spectrum. Although a particular precursor of approximately known mass within the isolation window may be specifically "targeted", the present analysis assumes that the isolation window may contain any ion whose mass is in the isolation window-whether seen or unseen in the MS$^1$ spectrum. In fact, it is assumed that the isolation window includes a linear combination of all masses in the range for which a local mass spectral database entry is available. Therefore, the observed product ion spectrum is assumed to be a linear combination of the corresponding fragmentation spectra in the database. Note that, in practice, one must also accommodate the possibility that ions of multiple charge state and isotope composition may also fall in the isolation window. This is a technical detail that does not change the general form of the algorithm.

Suppose that there are *K* candidate product ion spectra in the database corresponding to a given precursor isolation window. A database matrix **D** containing *N* rows and *K* columns is constructed as shown in Eq. 1,

$$\mathbf{D} = \begin{bmatrix} \mathbf{d}_1 & \mathbf{d}_2 \cdots \mathbf{d}_K \end{bmatrix} = \begin{bmatrix} d_{1,1} & d_{1,2} & \cdots & d_{1,K} \\ d_{2,1} & d_{2,2} & \cdots & d_{2,K} \\ \vdots & \vdots & \ddots & \vdots \\ d_{N,1} & d_{N,2} & \cdots & d_{N,K} \end{bmatrix} = \begin{bmatrix} d_{n,k} \end{bmatrix}_{N \times K} \qquad (1)$$

in which each column, $\mathbf{d}_k$ (for $k=1...K$) is a database (spectral library) spectrum represented as a (column) vector having $N$ entries, $d_{n,k}$ (for $n=1...N$). The entry $d_{n,k}$ is the observed intensity of the $k^{th}$ product ion spectrum at the $n^{th}$ m/z "position", this intensity possibly being an integrated intensity over a narrow mass range. It is necessary that the product ion spectra be normalized so that the entries at corresponding positions in the vector represent equivalent mass positions.

Once again supposing that s denotes a product ion spectrum, considered a vector, it is desired to interpret in terms of the spectral library **D**. In particular, it is desired to find the weighted sum of database spectra that most closely approximates **s.** An arbitrary linear combination, **s',** of the database spectra is given by Eq. 2, viz.

$$\mathbf{s}' = \sum_{k=1}^{K} \beta_k ' \mathbf{d}_k = \begin{bmatrix} \mathbf{d}_1 & \mathbf{d}_2 \cdots \mathbf{d}_K \end{bmatrix} \begin{bmatrix} \beta_1' \\ \beta_2' \\ \vdots \\ \beta_K' \end{bmatrix} = \mathbf{D} \, \mathbf{x}' \qquad (2)$$

in which the column vector $\beta$' is an arbitrary vector of $K$ abundances, not necessarily optimal. The goal is to determine an optimal estimate of the abundances, which is denoted by $\widehat{\beta}$, where the product ion spectrum **s** is interpreted as a mixture of database spectra. According to the present model, $\widehat{\beta}$ is also an estimate of the abundances of the precursors, represented by these database spectra, that occur in the unknown mixture that gave rise to the observed product ion spectrum **s.** The optimality criterion used here is minimization of the sum of weighted squared differences between the components of **s** and **s',** denoted by the scalar quantity e in Eq. 3, below. The sum is over the data samples in the observed spectrum **s.** The quantity e is the squared length of the vector difference between **s** and **s'.** The squared length of a vector can be written as the transpose of a vector times itself.

$$e = \sum_{n=1}^{N} (s'_n - s_n)^2 = (\mathbf{s}' - \mathbf{s})^{\mathrm{T}} (\mathbf{s}' - \mathbf{s}) = (\mathbf{D}\mathbf{x}' - \mathbf{s})^{\mathrm{T}} (\mathbf{D}\mathbf{x}' - \mathbf{s}) \qquad (3)$$

Minimizing the squared length of the vector is equivalent to minimizing the length of the vector, and more convenient mathematically. Correspondence between the mass positions represented by the entries of **s** and the database product ion spectra so that a vector difference between the quantities is meaningful.

Determining the set of parameter values that minimizes the sum of squared differences between a model and observed data is equivalent to maximum-likelihood estimation in the special case where the observed data is assumed to be the outcome of a random process in which an "ideal" model is corrupted by additive, white Gaussian noise. As described in U.S. Pre-Grant Publ. No. 2014/0138535 A1, if weighting factors are all assumed to be unity, the solution vector $\widehat{\beta}$ that minimizes the sum of squares, e, is given by:

$$\widehat{\beta} = (\mathbf{D}^{\mathrm{T}}\mathbf{D})^{-1} \mathbf{D}^{\mathrm{T}} \mathbf{s} \qquad (4)$$

which is similar to the conventional least-squares formula, but with slightly different notation.

*Generation of Local Mass Spectral Library*

United States Pre-Grant publications 2014/0138535 A1 and 2014/0138535 A1 describe populating the entries of a local mass spectral library on a spectrum-by-spectrum basis, as new, previously un-tabulated spectral components are discovered. Although such a method may be successfully implemented, in practice, the newly discovered components must await later annotation in order to identify their amino sequence information. By contrast, methods in accordance with the present teachings utilize conventional database searches to initially seed and annotate the local mass spectral library.

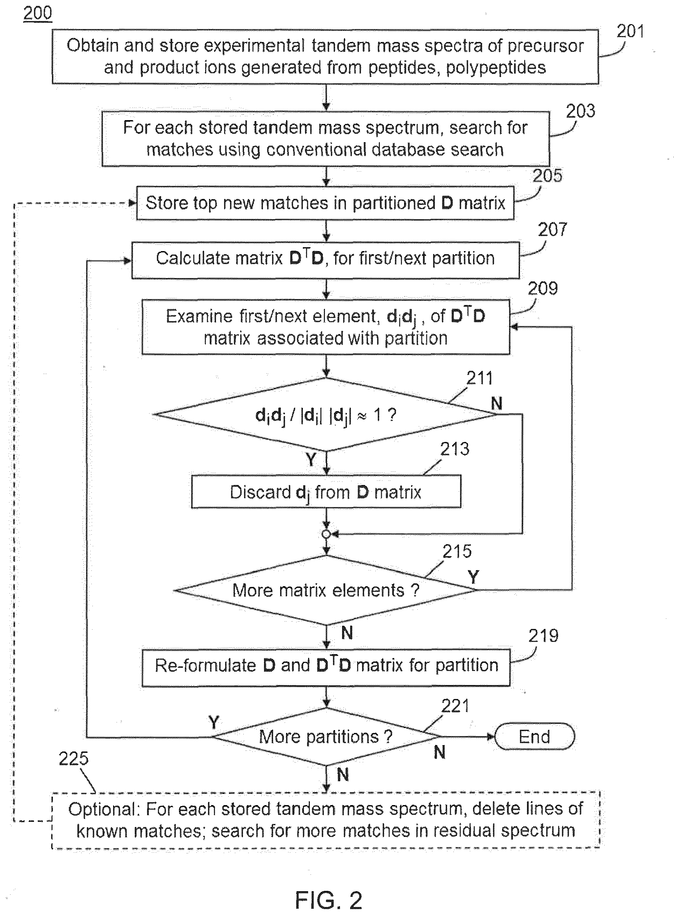

An exemplary such method for generating an initial database is illustrated in FIG. 2 as method **200.** In the initial step **201** of the method **200,** so as to generate the data that will be used to initially populate the entries of the local mass spectral library, a particular mass spectrometer is operated in conventional fashion. In this step, the mass spectrometer detects and records the mass spectra of peptide and polypeptide compounds (possibly multiplexed) in mixtures of possibly unknown analytes. For example, one or more of the various mass spectra may be obtained from detection and measurement of ions formed from various compounds eluting from a liquid chromatograph. Other mass spectra may be obtained from ionization of pure compounds or mixtures that are infused into the mass spectrometer. The execution of the step **201** may require hours, days, weeks are longer, depending on the speed of preparation of samples and the rate at which the mass spectrometer is utilized. All mass spectra to be employed in the generation of a local mass spectral library are obtained using the same mass spectrometer instrument, preferably using consistent operational settings or parameters. Separate mass spectral libraries may correspond to spectral obtained using respective different sets of operational settings or parameters.

In step **203** of the method **200** (FIG. 2), a standard database search (for example using the SEQUEST or MASCOT programs) is conducted with regard to the stored data of each spectrum. The mass spectral lines corresponding to the top scoring matches are then entered into the local mass spectral library (step **205**). Either prior to or at the time of initial population of the library entries, a standard bin size must be chosen whereby the measured $m/z$ values are rounded before entry into the library and recorded in the library at the nearest bin increment. Such binning of the data is necessary because - even though $m/z$ values may occur within or over a virtual continuum of values, only a finite number, $N,$ of entries rows may be recorded in the **D** matrix (see Eq. 1). The bin width may be chosen according to the requirements of the user or according to instrumental factors, such as available mass spectral resolution. Accordingly, the observed intensity of each observed line - most often, a product-ion line - that corresponds to a recognized match is entered into the local mass spectral library at the $m/z$ value of the bin position nearest to the measured $m/z$ value and each set of lines that corresponds to a single database match is entered as a single respective column in the **D** matrix.

The matrix **D** may be partitioned into a plurality of sub-matrices. Each sub-matrix may correspond to a restricted $m/z$ range of precursor ions. The widths of the $m/z$ ranges corresponding to the various partitions may be - but are not necessarily - chosen according to considerations similar to those used to choose bin widths. The widths of the $m/z$ ranges corresponding to the partitions may be - but are not necessarily - identical. Partitioning the mass spectral data in this fashion simplifies subsequent matrix calculations, since each set of calculations is only performed with regard to the data within each partition. The data within each partition may be regarded as a sub-library of the data. Step **205** of the method **200** is the first step in a loop of steps, in which the data within each respective partition is considered during each iteration of the loop.

Steps **209-219** of the method **200** (FIG. 2) comprise quality control steps within which redundant mass spectral data is pruned from each partition. Since the calculation of Eq. 4 relies on the inverse of the $\mathbf{D}^T\mathbf{D}$ matrix, precautions must be taken to ensure that the determinant of this matrix is not zero. A zero determinant can indicate either that: (a) the partition sub-library contains redundant data, or (b) at least one column of the matrix is a linear combination of two or more other columns - that is, at least one column represents superimposed mass spectra. Since each column in the **D** matrix corresponds to a match to a computer-generated theoretical spectrum, it is here considered that case (b) can be ignored. Thus, instead of calculating the determinant of each $\mathbf{D}^T\mathbf{D}$ matrix, it is here assumed that it is only necessary to calculate and inspect the individual elements of each such matrix (steps **209** and **211** of method **200**). Let **A** denote the $K\times K$ matrix $\mathbf{D}^T\mathbf{D}$. The general entry $A_{i,j}$ of this matrix is equal to $\mathbf{d}_i^T\mathbf{d}_j$, or equivalently the dot product between database product ion spectra $\mathbf{d}_i$ and $\mathbf{d}_j$. If the ratio between this dot product and the product of the norms, $|\mathbf{d}_i|$ and $|\mathbf{d}_j|$ of the two vectors, $\mathbf{d}_i$ and $\mathbf{d}_j$, is approximately equal to unity, within a certain pre-determined increment (step **211**), then the two spectra are considered to be redundant and one of them is discarded from the **D** matrix in step **213.** The discarded vector may be either $\mathbf{d}_i$ or $\mathbf{d}_j$, but is shown as $\mathbf{d}_j$ in step **213.**

Step **215** of the method **200** is a loop termination step in which, if any more matrix elements of the $\mathbf{D}^T\mathbf{D}$ matrix remain unexamined, then execution branches back to step **209,** in which the next element is considered in turn. After all elements of the $\mathbf{D}^T\mathbf{D}$ matrix under consideration have been examined in the above fashion, the **D** and $\mathbf{D}^T\mathbf{D}$ matrices are reformulated in smaller size if any vectors have been discarded in step **213.** The step **221** is a loop termination step in which the next partition (if any) is set for consideration in the next execution of step **207.** Once all partitions have been considered, execution of the method **200** may end. Optionally, however, the set of stored mass spectra may be examined again for matches with the SEQUEST or MASCOT programs (step **225**), this second examination conducted after mass spectral lines corresponding to previously determined matches have been deleted from the spectrum. Such conventional database searching techniques often do not detect multiplexed spectra and only report the highest scoring match. By submitting the "synthetic" residual spectrum created by deleting previously recognized matches, additional matches can be recognized and entered into the library (re-execution of step **205** after step **225**). A second pass on the various fragment-ion spectra may result in the discovery of additional significant spectral matches corresponding to co-isolation of additional precursors within the partition window.

A local mass spectral library includes, at a minimum, the information as may be represented by a matrix **D** (see Eq. 1) in which each column, $\mathbf{d}_k$ (for $k$=1...$K$) is an idealized spectrum represented as a (column) vector having $N$ entries, $d_{n,k}$ (for $n$=1...$N$). The entry $d_{n,k}$ is the observed intensity of the $k^{\text{th}}$ such mass spectrum at the $n^{\text{th}}$ bin position, each bin position representing a particular $m/z$ ratio. Each column vector represents an idealized (as opposed to an actual) spectrum because each $m/z$ ratio is rounded to the nearest bin position and only line intensities above a certain threshold are included; "noise" is excluded. Any $m/z$ bin positions that do not correspond to lines are occupied by the value zero. In addition to the **D** matrix, the local mass spectral library may also contain other derived matrices (such as the $\mathbf{D}^{\text{T}}$ matrix and the $(\mathbf{D}^{\text{T}}\mathbf{D})^{-1}$ matrix; see Eq. 4) which may be precalculated and stored in the mass spectral library prior to performing regression calculations using the data of the library.

The local mass spectral library may be partitioned, such that each partition contains a sub-matrix (or a partition of a larger matrix) that relates only to ions that are: (a) product ions formed by fragmentation of precursor ions that are isolated within an isolation window and subsequently fragmented; and, optionally, (b) the precursor ions themselves. The isolation window corresponds to a range of $m/z$ values that may include more than one precursor ion, either intentionally or unintentionally, upon isolation. In this document, no distinction is made between a **D** matrix (or other matrix) that corresponds to all the column vectors in the local mass spectral library and a **D** matrix that is a sub-matrix that corresponds to a partition of a larger **D** matrix. Each partition may include its own **D** matrix and, optionally, its own $\mathbf{D}^{\text{T}}$ matrix and the $(\mathbf{D}^{\text{T}}\mathbf{D})^{-1}$ matrix. Of course, any time a **D** matrix is modified, such as by addition of new data, the corresponding derived matrices in the library or library partition must be recalculated.

Each column vector in the **D** matrix presumably corresponds to an individual analyte that has been detected and measured by the particular mass spectrometer system to which the local mass spectral library pertains. Each such idealized mass spectrum may be representative of any type of mass spectrum. However, most often, the **D** matrix will comprise column vectors that represent product ion spectra, each such product ion spectrum comprising information relating to product ions generated from a single precursor ion. In such cases, each column vector may contain additional entries that provide information relating to the $m/z$ ratio and measured spectral intensity of the precursor ion that generated the product ions. Practically, this corresponds to additional rows of the **D** matrix that provides the precursor information. Even if the **D** matrix in question is a sub-matrix that is part of a partition of a larger matrix, where the partition corresponds to a precursor ion isolation window, the isolation window may comprise a range of $m/z$ values within which multiple precursor ions may be co-isolated. In this case, the bin positions of the additional rows correspond to $m/z$ values within the isolation window.

A local mass spectral library (or one of its partitions) may contain ancillary textual or numeric information that is not directly involved in regression calculations employing the library. The ancillary information may include information pertaining to or derived from the conventional database search or searches employed during the construction of subsequent modification of the library. Such information may include, but is not necessarily limited to: the dates of creation or modification of the library; types and number of database searches employed; and analyte species identification, assignment information or sequence information for some or all of the analytes represented in the library. The ancillary information may include information pertaining to local input data used to create the library and may include, without limitation: the identities and measurement dates of locally generated mass spectra employed in the library creation or modification; analyst names; instrumental calibration information; number of $m/z$ bins, range of binned data and binning resolution; and instrumental operating parameters used during acquisition of the locally generated mass spectra. A library partition may include, without limitation, information relating to: precursor $m/z$ isolation window corresponding to the partition; and operational parameters relating to a collision or fragmentation cell.

*Searching raw data against the spectral library*

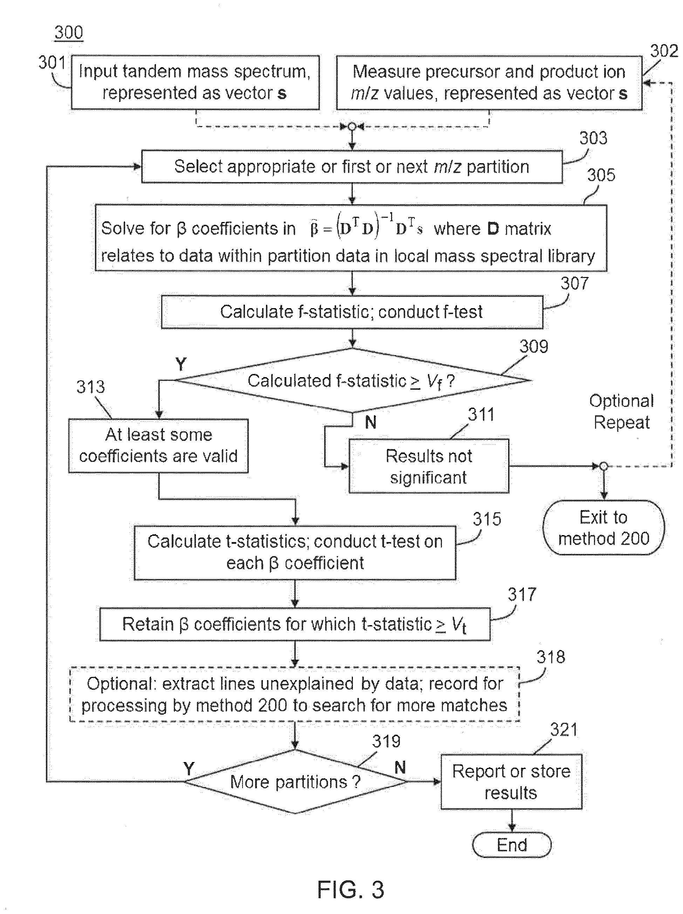

FIG. 3 is a flow diagram of an example method **300** for recognizing or identifying measured mass spectra. Once a high quality database has been generated, each newly generated spectrum may be represented by its vector, **s,** corresponding to the masses of its precursor and fragment ions (step **301 or step 302**). The vector, **s,** may be searched against the local database partition (step **303**) and the coefficients (representing spectra in the database) may be computed (step **305**) as given by Eq. 4. The vector $\widehat{\beta}$ is the set of estimator coefficients generated by the calculation, each of which corresponds to an individual spectrum (each spectrum represented as a column vector) in the local mass spectral library.

The method **300** may be performed "offline" subsequent to data acquisition - that is, using a previously generated spectrum file or files to provide the data which is input in step **301**. However, to save computational time, the $\mathbf{D}^T$ and $\mathbf{D}^T\mathbf{D}$ matrices may be pre-computed prior to executing the method **300**. Therefore, at least some steps of the method **300** may be performed "in real time", concurrently with the continued operation of the mass spectrometer that generated the data being processed by the steps of the method. In this latter situation, the data may be provided directly from the mass spectrometer, in the alternative step **302**. The use of a fast computational processor, such as a graphics processing unit (GPU) can enable execution of steps up to at least step **311** in real time. This can enable a form of operation in which subsequent operation of the mass spectrometer is made dependent on the results of the steps through step **311,** indicated as a dashed line leading back to step **302**.

Once the coefficients are computed an f-test is conducted to validate the model using a calculated f-statistic (step **307**). The f-test may be formulated in a number of different ways. One way of formulating the statistic is as an analysis of variance (ANOVA) f-statistic. Under such a formulation, a value of the f-statistic that is sufficiently large - specifically greater than a pre-determined value, $V_f$ as in step **309** - indicates that at least one or more coefficients are statistically significant (step **313**). Otherwise, the calculated regression coefficients are considered to be not statistically significant (step **311**). At this point, the method **300** may simply terminate without providing substantive results other than possibly reporting the failure to fit the data to the model. Alternatively, the method **300** may exit to method **200** (FIG. 2), since the failure to fit the new data as a combination of spectra already within the local mass spectral library may indicate the observation of spectrum not already present in the library. This spectrum can be searched using conventional database searching techniques (method **200**), annotated with a unique sequence and added to the library. As a still-further alternative, if the steps **303-311** are being performed in real time, the failure to find a statistically significant match to existing data may mean that a different precursor ion should be selected or isolated for fragmentation in order to identify an analyte. Thus, in this case, the method **300** branches back to step **302,** as shown by the dashed line, at which the different precursor ion is fragmented, and the new data is submitted for processing.

After completion of an f-test, one or more t-tests (step **315**) can be conducted to tease out any other spectrum in the isolation window. A t-test may be conducted for each regression coefficient to test the null hypothesis that the respective coefficient is zero - that is, the coefficient does not statistically explain the data variation. Those coefficients for which statistical significance is indicated are retained or stored for future reporting in step **317**. Statistical significance is indicated by the t-statistic exceeding some pre-determined value $V_t$. Furthermore, unassigned fragment ions of significant signal intensity can be extracted from the observed spectrum and stored as a separate file (step **318**) which can be later researched as, for example, according to the method **200** (FIG, 2). If the measured spectral data encompasses more than one partition of the local mass spectral library and if not all such partitions have been considered (step **319**), then the method **300** branches back to step **303**. Otherwise the calculated results are reported or stored in step **321**. The reporting or storing may include one or more of: observed line positions and intensities; reference identifications of library spectra matched; regression coefficients; other calculated statistics; annotation data (such as amino acid sequences) of the matched library data and positions of unmatched lines.

## Claims

1. A method for generating a partitioned local mass spectral library for a mass spectrometer, the method comprising measuring a plurality of experimental tandem mass spectra of peptides and polypeptides using the mass spectrometer and storing the tandem mass spectra in one or more data files, wherein each tandem mass spectrum includes a respective product-ion mass spectrum, the method **CHARACTERIZED BY**:

   (a) for each tandem mass spectrum stored in the data file:

   (a1) searching a standard database comprising a list of computer-generated theoretical product-ion mass spectra for a match or matches to one or more mass spectral lines of the stored tandem mass spectrum, each match determined according to a score determination; and
   (a2) for each match, creating an entry within a partition of the local mass spectral library that includes the one or more mass spectral lines, wherein the entry is represented in the partition as a vector $\mathbf{d}_k$, defined as

$$\mathbf{d}_k = \begin{bmatrix} d_1 \\ d_2 \\ \vdots \\ d_N \end{bmatrix}_k = \left[ d_n \right]_k$$

where each entry $d_n$ corresponds to an $n^{th}$ mass-to-charge ($m/z$) position and is either zero or else an observed intensity of a single one of the measured one or more mass spectral lines at said $n^{th}$ position;

(b) for each partition of the local mass spectral library, performing the steps of:

(b1) for each unique pair ($\mathbf{d}_{k1}$, $\mathbf{d}_{k2}$) of entries in the partition, determining if information provided by $\mathbf{d}_{k2}$ is redundant to information provided by $\mathbf{d}_{k1}$; and
(b2) discarding either $\mathbf{d}_{k1}$ or $\mathbf{d}_{k2}$ and from the partition if information provided by $\mathbf{d}_{k2}$ is redundant to information provided by $\mathbf{d}_{k1}$; and

(c) for each tandem mass spectrum stored in the data file:

(c1) generating a respective residual mass spectrum by deleting from the stored tandem mass spectrum all mass spectral lines that correspond to an existing entry in the local mass spectral library;
(c2) searching the standard database for a match or matches to one or more mass spectral lines of the residual mass spectrum, each match determined according to a score determination; and
(c3) if one or more mass spectral lines of the residual mass spectrum match an entry in the standard database, creating an entry within a partition of the local mass spectral library that includes the one or more mass spectral lines of the residual mass spectrum.

**Patentansprüche**

1. Ein Verfahren zur Erzeugung einer partitionierten lokalen Massenspektralbibliothek für ein Massenspektrometer, wobei das Verfahren das Messen einer Vielzahl experimenteller Tandem-Massenspektra von Peptiden und Polypeptiden mithilfe des Massenspektrometers und das Speichern der Tandem-Massenspektra in einer oder mehreren Datendateien umfasst, wobei jedes Tandem-Massenspektrum ein entsprechendes Produkt-Ionen-Massenspektrum umfasst, und wobei das Verfahren **gekennzeichnet ist durch**:

(a) für jedes Tandem-Massenspektrum, das in der Datendatei gespeichert ist:

(a1) Durchsuchen einer Standard-Datenbank, die eine Liste von computergenerierten theoretischen Produkt-Ionen-Massenspektra umfasst, nach einer Übereinstimmung oder Übereinstimmungen für eine oder mehrere Massenspektrallinien des gespeicherten Tandem-Massenspektrums, wobei jede Übereinstimmung entsprechend einer Score-Ermittlung determiniert wird; und
(a2) für jede Übereinstimmung, das Erzeugen eines Eintrags innerhalb eines Teilbereichs der lokalen Massenspektralbibliothek, die die eine oder mehreren Massenspektrallinien umfasst,
wobei der Eintrag in dem Teilbereich als Vektor $\mathbf{d}_k$ dargestellt wird, definiert als

$$d_k = \begin{bmatrix} d_1 \\ d_2 \\ . \\ . \\ . \\ . \\ d_N \end{bmatrix}_k = [d_n]_k$$

wobei jeder Eintrag $d_n$ mit einer *n-ten* Masse-Ladungsverhältnis- *(m/z)* Position korrespondiert und entweder Null oder andernfalls eine beobachtete Intensität einer einzelnen der gemessenen einen oder mehreren Massenspektrallinien an besagter n-terPosition ist;

(b) für jeden Teilbereich der lokalen Massenspektralbibliothek das Durchführen der folgenden Schritte:

(b1) Ermitteln für jedes einmalige Paar ($\mathbf{d}_{k1}$, $\mathbf{d}_{k2}$) von Einträgen in dem Teilbereich, ob von $\mathbf{d}_{k2}$ bereitgestellte Informationen zu von $\mathbf{d}_{k1}$ bereitgestellten Informationen redundant sind; und
(b2) Verwerfen von entweder $\mathbf{d}_{k1}$ oder $\mathbf{d}_{k2}$ und von dem Teilbereich, falls von $\mathbf{d}_{k2}$ bereitgestellte Informationen zu von $\mathbf{d}_{k1}$ bereitgestellten Informationen redundant sind; und

(c) für jedes in der Datendatei gespeicherte Tandem-Massenspektrum:

(c1) Erzeugen eines entsprechenden Residual-Massenspektrums **durch** Löschen aller Massenspektrallinien von dem gespeicherten Tandem-Massenspektrum, die einem vorhandenen Eintrag in der lokalen Massenspektralbibliothek entsprechen;
(c2) Durchsuchen der Standard-Datenbank nach einer Übereinstimmung oder Übereinstimmungen mit einer oder mehreren Massenspektrallinien des Residual-Massenspektrums, wobei jede Übereinstimmung entsprechend einer Score-Ermittlung determiniert wird; und
(c3) falls eine oder mehrere Massenspektrallinien des Residual-Massenspektrums mit einem Eintrag in der Standard-Datenbank übereinstimmt, Erzeugen eines Eintrags innerhalb eines Teilbereichs der lokalen Massenspektralbibliothek, der die eine oder mehreren Massenspektrallinien des Residual-Massenspektrums beinhaltet.

**Revendications**

1. Procédé de génération d'une spectrotèque de masse locale partitionnée pour un spectromètre de masse, le procédé comprenant la mesure d'une pluralité de spectres de masse en tandem expérimentaux de peptides et de polypeptides à l'aide du spectromètre de masse et le stockage de spectres de masse en tandem dans un ou plusieurs fichiers de données, dans lequel chaque spectre de masse en tandem comporte un spectre de masse d'ion produit respectif, le procédé étant **caractérisé par** :

(a) pour chaque spectre de masse en tandem stocké dans le fichier de données :

(a1) la recherche dans une base de données type comprenant une liste de spectres de masse d'ion produit théoriques générés par ordinateur d'une ou de concordance(s) avec une ou plusieurs raies spectrales de masse du spectre de masse en tandem stocké, chaque concordance étant déterminée selon une détermination de résultat ; et
(a2) pour chaque concordance, la création d'une entrée au sein d'une partition de la spectrotèque de masse locale qui comporte une ou plusieurs raies spectrales de masse, dans lequel l'entrée est représentée dans la partition en tant que vecteur $d_k$, définie sous la forme

$$\mathbf{d}_k = \begin{bmatrix} d_1 \\ d_2 \\ \vdots \\ d_N \end{bmatrix}_k = [d_n]_k$$

où chaque entrée $d_n$ correspond à une $n^{\text{ième}}$ position de masse sur charge (*m/z*) et est soit zéro soit sinon une intensité observée d'une seule des une ou plusieurs raies spectrales de masse mesurées à ladite $n^{\text{ième}}$ position ;

(b) pour chaque partition de la spectrotèque de masse locale, la réalisation des étapes de :

(b1) pour chaque paire unique ($d_{k1}$, $d_{k2}$) d'entrées dans la partition, le fait de déterminer si les informations

fournies par $d_{k2}$ sont redondantes par rapport à des informations fournies par $d_{k1}$ ; et
(b2) rejet soit de $d_{k1}$ soit de $d_{k2}$ et de la partition si des informations fournies par $d_{k2}$ sont redondantes par rapport à des informations fournies par $d_{k1}$ ; et

(c) pour chaque spectre de masse en tandem stocké dans le fichier de données :

(c1) la génération d'un spectre de masse résiduel respectif en supprimant du spectre de masse en tandem stocké toutes les raies spectrales de masse qui correspondent à une entrée existante dans la spectrotèque de masse locale ;
(c2) la recherche dans la base de données type d'une ou de concordance(s) avec une ou plusieurs raies spectrales de masse du spectre de masse résiduel, chaque concordance étant déterminée selon une détermination de résultat ; et
(c3) si une ou plusieurs raies spectrales de masse du spectre de masse résiduel concorde(nt) avec une entrée dans la base de données type, la création d'une entrée au sein d'une partition de la spectrotèque de masse locale qui comporte les une ou plusieurs raies spectrales de masse du spectre de masse résiduel.

FIG. 1A

50
54
52
56

FIG. 1B

61

FIG. 1C

52
62 62 62 62 62

FIG. 1D

54
64 64 64 64

FIG. 1E

56
66 66 66 66 66 66

200

Obtain and store experimental tandem mass spectra of precursor and product ions generated from peptides, polypeptides — 201

For each stored tandem mass spectrum, search for matches using conventional database search — 203

Store top new matches in partitioned **D** matrix — 205

Calculate matrix $\mathbf{D}^T\mathbf{D}$, for first/next partition — 207

Examine first/next element, $\mathbf{d_i}\mathbf{d_j}$, of $\mathbf{D}^T\mathbf{D}$ matrix associated with partition — 209

$\mathbf{d_i}\mathbf{d_j} / |\mathbf{d_i}| |\mathbf{d_j}| \approx 1$ ? — 211
N

Y

Discard $\mathbf{d_j}$ from **D** matrix — 213

More matrix elements ? — 215
Y

N

Re-formulate **D** and $\mathbf{D}^T\mathbf{D}$ matrix for partition — 219

More partitions ? — 221
Y

N → End

N

225

Optional: For each stored tandem mass spectrum, delete lines of known matches; search for more matches in residual spectrum

FIG. 2

300

301 Input tandem mass spectrum, represented as vector **s**

302 Measure precursor and product ion *m/z* values, represented as vector **s**

303 Select appropriate or first or next *m/z* partition

305 Solve for β coefficients in $\bar{\beta} = \left(\mathbf{D}^{\mathrm{T}}\mathbf{D}\right)^{-1}\mathbf{D}^{\mathrm{T}}\mathbf{s}$ where **D** matrix relates to data within partition data in local mass spectral library

307 Calculate f-statistic; conduct f-test

309 Calculated f-statistic $\geq V_f$ ?

Y

313 At least some coefficients are valid

N

311 Results not significant

Optional Repeat

Exit to method 200

315 Calculate t-statistics; conduct t-test on each β coefficient

317 Retain β coefficients for which t-statistic $\geq V_t$

318 Optional: extract lines unexplained by data; record for processing by method 200 to search for more matches

319 More partitions ?

Y

N

321 Report or store results

End

FIG. 3

**EP 3 002 696 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140138535 A1